(19) Europäisches Patentamt / European Patent Office / Office européen des brevets

(11) **EP 3 936 225 A1**

(12) **EUROPEAN PATENT APPLICATION**
published in accordance with Art. 153(4) EPC

(43) Date of publication:
**12.01.2022 Bulletin 2022/02**

(51) Int Cl.:
**B01J 20/26** *(2006.01)*          **A61F 13/49** *(2006.01)*
**A61F 13/53** *(2006.01)*          **B01J 20/28** *(2006.01)*

(21) Application number: **20770289.5**

(22) Date of filing: **05.03.2020**

(86) International application number:
**PCT/JP2020/009507**

(87) International publication number:
**WO 2020/184394 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2019 JP 2019042881**

(71) Applicant: **Sumitomo Seika Chemicals Co., Ltd.
Kako-gun, Hyogo 675-0145 (JP)**

(72) Inventor: **NISHIDA, Moe
Himeji-shi, Hyogo 6728076 (JP)**

(74) Representative: **Nederlandsch Octrooibureau
P.O. Box 29720
2502 LS The Hague (NL)**

(54) **WATER ABSORBENT RESIN PARTICLE, ABSORBER, ABSORBENT ARTICLE, METHOD FOR MEASURING PERMEATION RETENTION RATE OF WATER ABSORBENT RESIN PARTICLE, AND METHOD FOR PRODUCING WATER ABSORBENT RESIN PARTICLE**

(57)    Water-absorbent resin particles having a feeding retention rate of 10% or more are disclosed, the feeding retention rate being represented by the following formula using a feeding rate through a dry powder measured by a method including the steps (A), (B), and (C) in this order, and a feeding rate through a swollen gel measured by a method including the steps (A), (B), (D), and (E) in this order.

$$\text{Feeding retention rate (\%)} = (\text{feeding rate through swollen gel}/\text{feeding rate through dry powder}) \times 100$$

**EP 3 936 225 A1**

# Fig.3

**Description**

**Technical Field**

The present invention relates to water-absorbent resin particles, an absorber, an absorbent article, a method for measuring a feeding retention rate of water-absorbent resin particles, and a method for producing water-absorbent resin particles.

**Background Art**

A water-absorbent resin is used in the field of sanitary products. Specifically, it is used as a material for an absorber contained in an absorbent article such as a diaper (for example, Patent Literature 1).

**Citation List**

**Patent Literature**

[Patent Literature 1] Published Japanese Translation No. 2014-515987 of the PCT International Publication

**Summary of Invention**

**Technical Problem**

Absorbent articles such as diapers have an absorber that absorbs and retains body fluids such as urine and menstrual blood. The absorber generally contains water-absorbent resin particles and fibrous substances such as pulp, where the fibrous substances are entwined with each other, or the fibrous substances and the water-absorbent resin particles are entwined with each other. In recent years, since absorbent articles tend to be made thinner, absorbent articles in which a proportion of fibrous substances in an absorber is low have tended to be preferred. In a case where the proportion of the fibrous substances in the absorber is low, and when a shearing force is applied to the absorber after it absorbs a liquid due to, for example, a load of body weight and the like while an absorbent article is worn, deformation of the absorber such as tearing of the absorber from its inside may occur. The absorber in which tearing has been occurred cannot sufficiently exhibit its absorption performance.

An object of the present invention is to provide water-absorbent resin particles capable of inhibiting occurrence of tearing of an absorber after liquid absorption.

**Solution to Problem**

Water-absorbent resin particles of the present invention are water-absorbent resin particles having a feeding retention rate of 10% or more, the feeding retention rate being represented by the following formula using a feeding rate through a dry powder measured by a method including the following (A), (B), and (C) in this order, and a feeding rate through a swollen gel measured by a method including the following (A), (B), (D), and (E) in this order.

$$\text{Feeding retention rate (\%)} = (\text{feeding rate through swollen gel/feeding rate through dry powder}) \times 100$$

(A) 0.3 g of the water-absorbent resin particles is uniformly dispersed in a cylindrical container with an inner diameter of 26 mm having a mesh-like bottom part.

(B) A measurement unit is formed by inserting a cylindrical small-sized container with an outer diameter of 25 mm and an inner diameter of 19 mm having a mesh-like bottom part into the cylindrical container.

(C) A feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding.

(D) The water-absorbent resin particles in the measurement unit are swollen by immersing the bottom part side of the measurement unit in 40 g of physiological saline in the container for 30 minutes.

(E) A feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

In the water-absorbent resin particles, the feeding rate through a swollen gel is preferably 2.5 g/min or more. When the feeding rate through a swollen gel of the water-absorbent resin particles is within the above range, it is possible to further inhibit occurrence of tearing of an absorber after liquid absorption.

The present invention further provides an absorber containing the water-absorbent resin particles. When the absorber contains the water-absorbent resin particles, occurrence of tearing can be inhibited.

The present invention still further provides an absorbent article containing the absorber.

The absorbent article may be a diaper.

The present invention still further provides a method for measuring a feeding retention rate of water-absorbent resin particles which is represented by the following formula, the method including: measuring a feeding rate through a dry powder by a method including the following (A), (B), and (C) in this order; and measuring a feeding rate through a swollen gel by a method including the following (A), (B), (D), and (E) in this order.

$$\text{Feeding retention rate (\%)} = (\text{feeding rate through swollen gel/feeding rate through dry powder}) \times 100$$

(A) 0.3 g of the water-absorbent resin particles is uniformly dispersed in a cylindrical container with an inner diameter of 26 mm having a mesh-like bottom part.

(B) A measurement unit is formed by inserting a cylindrical small-sized container with an outer diameter of 25 mm and an inner diameter of 19 mm having a mesh-like bottom part into the cylindrical container.

(C) A feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding.

(D) The water-absorbent resin particles in the measurement unit are swollen by immersing the bottom part side of the measurement unit in 40 g of physiological saline in the container for 30 minutes.

(E) A feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

By the above-described measuring method, it is possible to evaluate diffusibility of water-absorbent resin particles involved in tear resistance of an absorber.

The present invention still further provides a method for producing water-absorbent resin particles, the method including sorting out water-absorbent resin particles having the feeding retention rate of 10% or more, the feeding retention rate being measured by the above-described method. According to this production method, it is possible to obtain water-absorbent resin particles which have better liquid diffusibility and inhibit occurrence of tearing after liquid absorption when being used in an absorber.

The present invention still further provides a method for inhibiting occurrence of tearing of an absorber containing water-absorbent resin particles after the absorber absorbs a liquid, the method including increasing the feeding retention rate of the water-absorbent resin particles which is measured by the above-described method.

**Advantageous Effects of Invention**

According to the present invention, water-absorbent resin particles capable of inhibiting occurrence of tearing of an absorber after liquid absorption are obtained.

**Brief Description of Drawings**

Fig. 1 is a cross-sectional view showing an example of an absorbent article.

Fig. 2 is a plan view showing an outline of a stirring blade (flat plate blade having slits on a flat plate portion) used in Examples.
Fig. 3 is a schematic cross-sectional view showing a method for measuring a feeding retention rate.
Fig. 4 is a schematic view showing a method for measuring non-pressurization DW.
Fig. 5 is a schematic cross-sectional view showing a method for measuring an absorber tearing time.

**Description of Embodiments**

Hereinafter, suitable embodiments of the present invention will be described in detail. However, the present invention is not limited to the following embodiments.

In the present specification, "acrylic" and "methacrylic" are collectively referred to as "(meth)acrylic." "Acrylate" and "methacrylate" are also referred to as "(meth)acrylate." "(Poly)" means both of a case where there is a prefix of "poly" and a case where there is no prefix thereof. Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified. In the present specification, physiological saline refers to an aqueous solution of NaCl at a concentration of 0.9% by mass unless otherwise specified.

In water-absorbent resin particles according to the present embodiment, a feeding retention rate represented by the following formula is 10% or more. The inventor of the present invention have found that occurrence of tearing of an absorber after liquid absorption is more inhibited as ratio of a feeding rate through a swollen gel, which is a feeding rate through water-absorbent resin particles in a swollen state after liquid absorption, to a feeding rate through a dry powder, which is a feeding rate through water-absorbent resin particles in a dry state, becomes higher.

$$\text{Feeding retention rate (\%)} = (\text{feeding rate through swollen gel/feeding rate through dry powder}) \times 100$$

According to the presumption of the inventor of the present invention, it is thought that, when a feeding retention rate is 10% or more, the water-absorbent resin particles of the present invention can diffuse a liquid more uniformly in the inside of an absorber. In a case where a liquid is not uniformly diffused in the inside of an absorber, a portion that is extremely unlikely to be torn (a portion that hardly absorbs the liquid) and a portion that is extremely likely to be torn (a portion that absorbs a large amount of the liquid) are diffusely present. When a load such as body weight is applied to an absorber while an absorbent article is worn, the absorber is likely to be torn from this portion that is extremely likely to be torn. It is thought that the water-absorbent resin particles of the present invention can uniformly diffuse a liquid in the inside of an absorber, and it is thought that it is difficult for a portion, which is extremely likely to be torn, to be generated, and as a result, occurrence of tearing of the absorber is inhibited.

The feeding rate through a dry powder is measured by a method including the following steps (A), (B), and (C) in this order. The feeding rate through a swollen gel is measured by a method including the following steps (A), (B), (D), and (E) in this order. More specific details of the measurement methods will be described in Examples to be described later.

(A) 0.3 g of the water-absorbent resin particles is uniformly dispersed in a cylindrical container with an inner diameter of 26 mm having a mesh-like bottom part.
(B) A measurement unit is formed by inserting a cylindrical small-sized container with an outer diameter of 25 mm and an inner diameter of 19 mm having a mesh-like bottom part into the cylindrical container.
(C) A feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding.
(D) The water-absorbent resin particles in the measurement unit are swollen by immersing the bottom part side of the measurement unit in 40 g of physiological saline in the container for 30 minutes.
(E) A feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

In the water-absorbent resin particles according to the present embodiment, the feeding retention rate is preferably 12% or more, is more preferably 15% or more, 20% or more, 25% or more, or 30% or more, and is even more preferably 35% or more or 40% or more. The feeding retention rate of the water-absorbent resin particles according to the present embodiment may be, for example, 80% or less, 70% or less, 60% or less, 58% or less, 55% or less, or 50% or less.

In the water-absorbent resin particles according to the present embodiment, the feeding rate through a dry powder measured by the above-described method may be, for example, 2 g/min or more, 5 g/min or more, 8 g/min or more, 10 g/min or more, 15 g/min or more, 18 g/min or more, or 20 g/min or more. The feeding rate through a dry powder of the water-absorbent resin particles may be, for example, 50 g/min or less, 40 g/min or less, 35 g/min or less, 33 g/min or less, or 30 g/min or less.

In the water-absorbent resin particles according to the present embodiment, the feeding rate through a swollen gel measured by the above-described method may be, for example, 0.7 g/min or more, 1.0 g/min or more, 1.5 g/min or more, 2.0 g/min or more, 2.5 g/min or more, 3.0 g/min or more, 4.0 g/min or more, 5.0 g/min or more, 6.0 g/min or more, or 8.0 g/min or more. When the feeding rate through a swollen gel is within the above-mentioned range, occurrence of tearing of an absorber after liquid absorption tends to be more inhibited. The feeding rate through a swollen gel of the water-absorbent resin particles may be, for example, 30 g/min or less, 25 g/min or less, 20 g/min or less, 18 g/min or less, or 15 g/min or less. From these viewpoints, the feeding rate through a swollen gel is preferably 1 to 30 g/min, 3 to 25 g/min, or 5 to 20 g/min.

The water-absorbent resin particles according to the present embodiment can have a high water-absorbing ability with respect to physiological saline. A water retention amount of the water-absorbent resin particles for physiological saline may be, for example, 20 g/g or more, 23 g/g or more, 25 g/g or more, 28 g/g or more, 30 g/g or more, or 32 g/g or more, and may be, for example, 60 g/g or less, 55 g/g or less, 53 g/g or less, 50 g/g or less, 48 g/g or less, 45 g/g or less, 43 g/g or less, or 40 g/g or less. A water retention amount of the water-absorbent resin particles for physiological saline may be, for example, 20 to 60 g/g, 23 to 55 g/g, 25 to 50 g/g, 28 to 45 g/g, or 30 to 40 g/g. The water retention amount for physiological saline is measured by a method described in Examples to be described later.

In the water-absorbent resin particles according to the present embodiment, a value of non-pressurization DW after 3 minutes may be, for example, 14 ml/g or more, 16 ml/g or more, 18 ml/g or more, 20 ml/g or more, or 25 ml/g or more. A value of non-pressurization DW after 3 minutes may be, for example, 55 ml/g or less, 50 ml/g or less, or 40 ml/g or less. The value of non-pressurization DW after 3 minutes is measured by a method described in Examples to be described later.

Examples of shapes of the water-absorbent resin particles include a substantially spherical shape, a crushed shape, and a granular shape. A median particle size of the water-absorbent resin particles may be 250 to 850 $\mu$m, 300 to 700 $\mu$m, or 300 to 600 $\mu$m. The water-absorbent resin particles according to the present embodiment may have a desired particle size distribution at a timing at which polymer particles are obtained by a production method to be described later, but their particle size distribution may be adjusted by performing operations such as adjustment of a particle size through classification with a sieve.

The water-absorbent resin particles according to the present embodiment can contain, for example, a crosslinking polymer formed by polymerization of monomers including ethylenically unsaturated monomers. The crosslinking polymer has a monomer unit derived from an ethylenically unsaturated monomer. That is, the water-absorbent resin particles according to the present embodiment can have a structural unit derived from ethylenically unsaturated monomers.

Examples of methods for polymerizing the monomers include a reverse phase suspension polymerization method, an aqueous solution polymerization method, a bulk polymerization method, and a precipitation polymerization method. Among them, the reverse phase suspension polymerization method or the aqueous solution polymerization method is preferable from the viewpoints of facilitating securement of favorable water-absorbent characteristics of the obtained water-absorbent resin particles and control of a polymerization reaction. Hereinbelow, a method for polymerizing ethylenically unsaturated monomers will be described with the reverse phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. A functional group such as a carboxyl group and an amino group, which is contained in the monomer, can function as a crosslinkable functional group in a surface crosslinking process to be described later. These ethylenically unsaturated monomers may be used alone or in a combination of two or more kinds thereof.

Among them, from the viewpoint of high industrial availability, the ethylenically unsaturated monomer preferably includes at least one compound selected from the group consisting of (meth)acrylic acid and a salt thereof, acrylamide, methacrylamide, and N,N-dimethyl acrylamide, and more preferably includes at least one compound selected from the group consisting of (meth)acrylic acid and a salt thereof, and acrylamide. The ethylenically unsaturated monomer more preferably includes at least one compound selected from the group consisting of (meth)acrylic acid and a salt thereof from the viewpoint of further enhancing water-absorbent characteristics.

For the monomer, a monomer other than the above-mentioned ethylenically unsaturated monomers may be partially used. Such a monomer can be used by, for example, being mixed with an aqueous solution containing the ethylenically unsaturated monomers. A use amount of the ethylenically unsaturated monomer may be 70 to 100 mol%, 80 to 100 mol%, 90 to 100 mol%, 95 to 100 mol%, or 100 mol%, with respect to a total amount of the monomers (the total amount of the monomers for obtaining the water-absorbent resin particles, for example, a total amount of monomers that gives a structural unit of a crosslinking polymer, the same shall apply hereinafter). Among them, (meth)acrylic acid and a salt thereof may be 70 to 100 mol%, 80 to 100 mol%, 90 to 100 mol%, 95 to 100 mol%, or 100 mol%, with respect to a total amount of monomers. The "proportion of (meth)acrylic acid and a salt thereof' means a proportion of a total amount of (meth)acrylic acid and a salt thereof.

According to the present embodiment, as an example of the water-absorbent resin particles, it is possible to provide water-absorbent resin particles which contain a crosslinking polymer having a structural unit derived from an ethylenically unsaturated monomer, in which the ethylenically unsaturated monomer includes at least one compound selected from the group consisting of (meth)acrylic acid and a salt thereof, and a proportion of (meth)acrylic acid and a salt thereof is 70 to 100 mol% with respect to a total amount of monomers for obtaining the water-absorbent resin particles.

Usually, the ethylenically unsaturated monomers are suitably used in a form of an aqueous solution. In general, it is sufficient for a concentration of the ethylenically unsaturated monomers in an aqueous solution containing the ethylenically unsaturated monomers (hereinafter, referred to as a monomer aqueous solution) to be 20% by mass or more and a saturated concentration or less, and it is preferably 25% to 70% by mass, and is more preferably 30% to 55% by mass. Examples of water to be used include tap water, distilled water, and ion-exchanged water.

In a case where ethylenically unsaturated monomers to be used have an acid group, a monomer aqueous solution may be used after neutralizing this acid group with an alkaline neutralizing agent. From the viewpoint of increasing an osmotic pressure of the obtained water-absorbent resin particles and thereby further enhancing water-absorbent characteristics such as a water retention amount, a degree of neutralization in the ethylenically unsaturated monomers by the alkaline neutralizing agent is 10 to 100 mol%, is preferably 50 to 90 mol%, and is more preferably 60 to 80 mol% of the acid group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution to simplify a neutralizing operation. The above-mentioned alkaline neutralizing agents may be used alone or in combination of two or more kinds thereof. Neutralization of the acid groups in the ethylenically unsaturated monomers can be performed by, for example, adding an aqueous solution of sodium hydroxide, potassium hydroxide, or the like dropwise to the monomer aqueous solution and mixing them.

In the reverse phase suspension polymerization method, a monomer aqueous solution is dispersed in a hydrocarbon dispersion medium in the presence of a surfactant, and polymerization of ethylenically unsaturated monomers is performed using a radical polymerization initiator or the like.

Examples of surfactants include nonionic surfactants and anionic surfactants. Examples of nonionic surfactants include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Among them, the surfactant preferably includes at least one compound selected from the group consisting of sorbitan fatty acid esters, polyglycerin fatty acid esters, and sucrose fatty acid esters, from the viewpoints that then, a state of a W/O type reverse-phase suspension becomes favorable, water-absorbent resin particles are likely to be obtained with suitable particle sizes, and industrial availability becomes high. Furthermore, the surfactant more preferably includes sucrose fatty acid esters from the viewpoint that water-absorbent characteristics of the obtained water-absorbent resin particles are then improved. These surfactants may be used alone or in combination of two or more kinds thereof.

An amount of the surfactant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these use amounts, and these amounts are economic.

Furthermore, a polymeric dispersant may be used in combination with the above-mentioned surfactant. Examples of polymeric dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. Among these polymeric dispersants, particularly from the viewpoint of dispersion stability of monomers, it is preferable to use maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer. These polymeric dispersants may be used alone or in combination of two or more kinds thereof.

An amount of the polymeric dispersant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these use amounts, and these amounts are economic.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). Among them, potassium persulfate, ammonium persulfate, sodium persulfate, 2,2' -azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride are preferred. These radical polymerization initiators may be used alone or in combination of two or more kinds thereof.

A use amount of the radical polymerization initiator may be 0.00005 to 0.01 moles with respect to 1 mole of the ethylenically unsaturated monomers. A case in which a use amount of the radical polymerization initiator is 0.00005 moles or more is efficient, because then a polymerization reaction is not required to be performed for a long period of time. In a case where a use amount thereof is 0.01 moles or less, a rapid polymerization reaction is unlikely to occur.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a chain transfer agent may be contained in an aqueous solution of the ethylenically unsaturated monomers used for the polymerization. Examples of chain transfer agents include hypophosphites, thiols, thiolic acids, secondary alcohols, and amines.

Furthermore, a thickener may be contained in the aqueous solution of the ethylenically unsaturated monomers used for the polymerization to control a particle size of the water-absorbent resin particles. As the thickener, it is possible to use, for example, hydroxyethyl cellulose, hydroxypropyl cellulose, methyl cellulose, carboxymethyl cellulose, polyacrylic acid, a (partially) neutralized product of polyacrylic acid, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the aqueous solution of the ethylenically unsaturated monomers becomes high.

Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. The hydrocarbon dispersion medium may include at least one compound selected from the group consisting of a chained aliphatic hydrocarbon having 6 to 8 carbon atoms and an alicyclic hydrocarbon having 6 to 8 carbon atoms. For the hydrocarbon dispersion medium, n-heptane, cyclohexane, or both n-heptane and cyclohexane may be contained, from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A use amount of the hydrocarbon dispersion medium is preferably 30 to 1,000 parts by mass, is more preferably 40 to 500 parts by mass, and is even more preferably 50 to 300 parts by mass, with respect to 100 parts by mass of the monomer aqueous solution, from the viewpoint that polymerization heat is then appropriately removed, and thereby a polymerization temperature is easily controlled. In a case where a use amount of the hydrocarbon dispersion medium is 30 parts by mass or more, there is a tendency that it becomes easy to control a polymerization temperature. In a case where a use amount of the hydrocarbon dispersion medium is 1,000 parts by mass or less, productivity of polymerization tends to be improved, which is economical.

In general, internal crosslinking may occur by self-crosslinking upon the polymerization, and internal crosslinking may be carried out by further using an internal crosslinking agent, and thereby water-absorbent characteristics of the water-absorbent resin particles may be controlled. Examples of internal crosslinking agents to be used include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. Among these internal crosslinking agents, it is preferable to use a polyglycidyl compound, it is more preferable to use a diglycidyl ether compound, and it is particularly preferable to use (poly)ethylene glycol diglycidyl ether, (poly)propylene glycol diglycidyl ether, and (poly)glycerin diglycidyl ether. These crosslinking agents may be used alone or in combination of two or more kinds thereof.

An amount of the internal crosslinking agent is preferably 0 to 0.03 moles, is more preferably 0.00001 to 0.01 moles, and is even more preferably 0.00002 to 0.005 moles, per 1 mole of the ethylenically unsaturated monomer, from the viewpoints of inhibiting water-soluble properties by appropriately crosslinking the obtained polymer, and exhibiting a sufficient water absorption amount.

An aqueous phase containing components such as an ethylenically unsaturated monomer, a radical polymerization initiator, and if necessary, an internal crosslinking agent; and an oil phase containing components such as a hydrocarbon dispersion medium, a surfactant, and if necessary, and a polymeric dispersant can be mixed and heated under stirring to carry out reverse phase suspension polymerization in a water-in-oil system.

When performing the reverse phase suspension polymerization, a monomer aqueous solution which contains ethylenically unsaturated monomers is dispersed in a hydrocarbon dispersion medium in the presence of a surfactant and if necessary, a polymeric dispersant. In this case, a timing of adding the surfactant or the polymeric dispersant before the start of the polymerization reaction may be either before or after the addition of the monomer aqueous solution.

Among them, it is preferable to carry out the polymerization after dispersing the monomer aqueous solution in the hydrocarbon dispersion medium in which the polymeric dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

Such reverse phase suspension polymerization can be carried out in one stage or in multiple stages of two or more stages. Furthermore, it is preferably carried out in two or three stages from the viewpoint of increasing productivity.

In a case where reverse phase suspension polymerization is carried out in multiple stages of two or more stages, it is sufficient for stages after a second stage of reverse phase suspension polymerization to be carried out in the same manner as in a first stage of reverse phase suspension polymerization by adding ethylenically unsaturated monomers to a reaction mixture obtained in the first stage of polymerization reaction and mixing them, after performing the first stage of reverse phase suspension polymerization. In reverse phase suspension polymerization in each stage after the second stage, it is preferable to carry out reverse phase suspension polymerization by adding, in addition to ethylenically unsaturated monomers, the above-mentioned radical polymerization initiator and internal crosslinking agent within a range of molar ratios of the respective components to the ethylenically unsaturated monomers, based on an amount of ethylenically unsaturated monomers added during reverse phase suspension polymerization in each stage after the second stage. If necessary, the internal crosslinking agent may be used in reverse phase suspension polymerization in each stage after the second stage. In a case where the internal crosslinking agent is used, it is preferable to carry out reverse phase suspension polymerization by adding the internal crosslinking agent within a range of molar ratios of the respective components to the ethylenically unsaturated monomers based on an amount of ethylenically unsaturated monomers provided in each stage.

A temperature for the polymerization reaction varies depending on radical polymerization initiators used, and it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrogel.

After the polymerization, post-polymerization crosslinking may be carried out by adding a crosslinking agent to the obtained hydrous gel polymer and heating them. By performing the post-polymerization crosslinking, a degree of crosslinking of the hydrous gel polymer can be increased, and thereby water-absorbent characteristics can be more preferably improved.

Examples of crosslinking agents for performing the post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and α-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. These crosslinking agents may be used alone or in combination of two or more kinds thereof.

An amount of the crosslinking agent used for the post-polymerization crosslinking is preferably 0 to 0.03 moles, is more preferably 0 to 0.01 moles, and is even more preferably 0.00001 to 0.005 moles, per 1 mole of the ethylenically unsaturated monomer, from the viewpoint of exhibiting suitable water-absorbent characteristics by appropriately crosslinking the obtained hydrous gel polymer.

It is sufficient for a timing for adding the post-polymerization crosslinking to be after polymerization of ethylenically unsaturated monomers used for the polymerization. In a case of multi-stage polymerization, the crosslinking agent is preferably added after the multi-stage polymerization. From the viewpoint of a water content (to be described later), it is preferable to add the crosslinking agent for the post-polymerization crosslinking within a region of [water content immediately after polymerization ± 3% by mass], in consideration of heat generation during and after polymerization, retention due to process delay, system opening when a crosslinking agent is added, and fluctuation in water content due to addition of water associated with addition of a crosslinking agent.

Subsequently, drying is performed to remove water from the obtained hydrous gel polymer. By drying, polymer particles containing the polymer of ethylenically unsaturated monomers are obtained. Examples of drying methods include a method (a) in which the hydrous gel polymer in a state of being dispersed in a hydrocarbon dispersion medium is subjected to azeotropic distillation by heating from the outside, and the hydrocarbon dispersion medium is refluxed to remove water; a method (b) in which the hydrous gel polymer is taken out by decantation and dried under reduced pressure; and a method (c) in which the hydrous gel polymer is separated by filtration with a filter and dried under reduced pressure. Among them, the method (a) is preferably used for its simplicity in production steps.

Control over a particle size of the water-absorbent resin particle can be performed, for example, by adjusting a rotation speed of a stirrer during the polymerization reaction or by adding a powdery inorganic flocculating agent to the system after the polymerization reaction or at an initial time of drying. A particle size of the obtained water-absorbent resin particle can be increased by adding the flocculating agent. Examples of powdery inorganic flocculating agents include silica, zeolite, bentonite, aluminum oxide, talc, titanium dioxide, kaolin, clay, and hydrotalcite. Among them, silica, aluminum oxide, talc, or kaolin is preferable from the viewpoint of a flocculation effect.

In the reverse phase suspension polymerization, the following method is preferable as a method of adding the powdery inorganic flocculating agent: a method in which a powdery inorganic flocculating agent is dispersed in a hydrocarbon dispersion medium of the same kind as that used in the polymerization, or water in advance, and then the mixture is mixed into a hydrocarbon dispersion medium containing a hydrogel under stirring.

In the production of the water-absorbent resin particles according to the present embodiment, a surface portion of the hydrous gel polymer is preferably crosslinking (surface crosslinked) using a crosslinking agent in the drying process or any of subsequent processes. The surface crosslinking is preferably performed at a timing when the hydrous gel polymer has a specific water content. A timing of the surface crosslinking is preferably a time point at which a water content of the hydrous gel polymer is 5% to 50% by mass, is more preferably a time point at which a water content thereof is 10% to 40% by mass, and is even more preferably a time point at which a water content thereof is 15% to 35% by mass.

A water content (% by mass) of the hydrous gel polymer is calculated by the following formula.

$$\text{Water content} = [\text{Ww}/(\text{Ww} + \text{Ws})] \times 100$$

Ww: An amount of water of a hydrous gel polymer obtained by adding an amount of water used, as desired, upon mixing a powdery inorganic flocculating agent, a surface crosslinking agent, and the like to an amount obtained by subtracting an amount of water extracted to the outside of the system by the drying process from an amount of water contained in an aqueous liquid before polymerization in the all polymerization processes.

Ws: A solid fraction calculated from an amount of materials introduced, such as ethylenically unsaturated monomers, a crosslinking agent, and an initiator, each of which constitutes the hydrous gel polymer.

Examples of surface crosslinking agents for performing surface crosslinking include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These surface crosslinking agents may be used alone or in combination of two or more kinds thereof.

In general, an amount of the surface crosslinking agent is preferably 0.00001 to 0.02 moles, is more preferably 0.00005 to 0.01 moles, and is even more preferably 0.0001 to 0.005 moles in a molar ratio, with respect to 1 mole of the ethylenically unsaturated monomer used in the polymerization, from the viewpoint of exhibiting suitable water-absorbent characteristics by appropriately crosslinking the obtained hydrous gel polymer.

A use amount of the surface crosslinking agent is preferably 0.00001 moles or more from the viewpoint of sufficiently increasing a crosslinking density in a surface portion of the polymer particles and thereby enhancing gel strength of the water-absorbent resin particles. Furthermore, a use amount thereof is preferably 0.02 moles or less from the viewpoint of increasing a water retention ability of the water-absorbent resin particles.

It is possible to obtain polymer particles, which are a surface crosslinked dried product, by distilling off water and the hydrocarbon dispersion medium by a known method after the surface crosslinking reaction.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, and the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly carry out the crosslinking reaction in polymer particles, and a feeding retention rate is easily adjusted within a range suitable for the invention of the present application while maintaining water-absorbent characteristics such as a water retention amount.

The water-absorbent resin particles according to the present embodiment may be composed of only the polymer particles, but they can further contain, for example, various additional components selected from inorganic powders, surfactants, oxidizers, reducing agents, metal chelating agents, radical chain inhibitors, antioxidants, antibacterial agents, deodorants, gel stabilizers, flowability improvers (lubricants), and the like. The additional components may be disposed inside the polymer particles, on a surface of the polymer particles, or both of the inside and on the surface thereof.

The water-absorbent resin particles according to the present embodiment preferably contain inorganic particles. Examples of inorganic particles include silica particles such as amorphous silica. The amorphous silica may be hydrophilic amorphous silica. The inorganic particles can be disposed on the surface of the polymer particles by, for example, mixing the polymer particles and the inorganic particles. The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size referred to herein can be a value measured by a dynamic light scattering method or a laser diffraction/scattering method. In a case where an amount of the inorganic particles added is within the above range, it is easy to obtain water-absorbent resin particles having favorable water-absorbent characteristics.

For example, flowability of the water-absorbent resin particles can be improved by adding 0.05 to 5 parts by mass of amorphous silica as inorganic particles with respect to 100 parts by mass of the polymer particles. In a case where the water-absorbent resin particles contain inorganic particles, a proportion of the inorganic particles with respect to a mass of the polymer particles may be 0.05% by mass or more, 0.1% by mass or more, 0.2% by mass or more, 0.5% by mass or more, 1.0% by mass or more, or 1.5% by mass or more, and may be 5.0% by mass or less, 3.5% by mass or less, 1.5% by mass or less, 1.0% by mass or less, 0.8% by mass or less, 0.5% by mass or less, or 0.3% by mass or less.

A method for producing water-absorbent resin particles according to the present embodiment may include a step of sorting out water-absorbent resin particles in which a feeding retention rate measured by the above-described method is 10% or more. The production method may include a step of measuring a feeding retention rate of the water-absorbent resin particles. As properties of the water-absorbent resin particles to be sorted out, water-absorbent resin particles, which satisfy the aspects (for example, a water retention amount for physiological saline within a specific range, a value of non-pressurization DW after 3 minutes within a specific range, and the like) of the above-described water-absorbent resin particles, may be sorted out.

The water-absorbent resin particles according to the present embodiment have better absorbency for body fluids such as urine and blood, and they can be applied to, for example, the fields of sanitary products such as paper diapers, sanitary napkins, and tampons, and animal excrement treatment materials such as pet sheets, and dog or cat litters.

The water-absorbent resin particles according to the present embodiment can be suitably used for an absorber. The absorber according to the present embodiment includes the above-mentioned water-absorbent resin particles. A content of the water-absorbent resin particles in the absorber is preferably 100 to 1,000 g per square meter of the absorber (that is, 100 to 1,000 g/m$^2$), is more preferably 150 to 800 g/m$^2$, and is even more preferably 200 to 700 g/m$^2$, from the viewpoint that sufficient liquid absorption performances are then obtained when the absorber is used for the absorbent article. A content thereof is preferably 100 g/m$^2$ or more from the viewpoint of exhibiting sufficient liquid absorption performances as the absorbent article, and thereby particularly inhibiting liquid leakage. A content thereof is preferably 1,000 g/m$^2$ or less from the viewpoint of inhibiting occurrence of a gel blocking phenomenon, and thereby exhibiting a diffusion performance of a liquid as the absorbent article and further improving a permeation rate of the liquid.

The absorber may further include, for example, a fibrous substance in addition to the water-absorbent resin particles. The absorber may be, for example, a mixture containing the water-absorbent resin particles and the fibrous substance. A mass proportion of the water-absorbent resin particles in the absorber may be 2% by mass to 100% by mass, is preferably 10% by mass to 80% by mass, and is more preferably 20% by mass to 70% by mass, with respect to a total of the water-absorbent resin particles and the fibrous substance. The configuration of the absorber may be, for example, a form in which water-absorbent resin particles and the fibrous substances are uniformly mixed, a form in which water-absorbent resin particles are held between fibrous substances formed in a sheet shape or a layer shape, or another form.

Examples of fibrous substances include finely pulverized wood pulp; cotton; cotton linter; rayon; cellulose-based fibers such as cellulose acetate; and synthetic fibers such as polyamides, polyesters, and polyolefins. In addition, the fibrous substance may be a mixture of the above-mentioned fibers.

Fibers may be adhered to each other by adding an adhesive binder to the fibrous substance in order to further enhance shape retention properties before or during use of the absorber. Examples of adhesive binders include heat-sealing synthetic fibers, hot melt adhesives, and adhesive emulsions. A use amount of an adhesive binder can be reduced since the water-absorbent resin particles according to the present embodiment are better in shape retainability when used in an absorber.

Examples of heat-sealing synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion is thermal bonded. Examples of hot melt adhesives include a blend of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone or in combination of two or more kinds thereof.

The absorber according to the present embodiment may further contain additives such as inorganic powders (for example, amorphous silica), deodorants, pigments, dyes, antibacterial agents, fragrances, and pressure sensitive adhesives. These additives can impart various functions to the absorber. In a case where the water-absorbent resin particles contain inorganic particles, the absorber may contain an inorganic powder in addition to the inorganic particles in the water-absorbent resin particles. Examples of inorganic powders include silicon dioxide, zeolite, kaolin, clay, and the like.

A shape of the absorber according to the present embodiment is not particularly limited, but it may be, for example, a sheet shape. A thickness of the absorber (for example, a thickness of a sheet-shaped absorber) may be, for example, 0.1 to 20 mm or 0.3 to 15 mm.

The absorbent article according to the present embodiment may include, for example, a core wrap, a liquid permeable top sheet, and a liquid impermeable back sheet, in addition to the absorber. The core wrap retains the shape of the absorber. The liquid permeable top sheet is disposed on the outermost part on a side from which an absorption target liquid is infiltrated. The liquid impermeable back sheet is disposed on the outermost part on a side opposite to the side from which the absorption target liquid is infiltrated.

Examples of the absorbent article include diapers (for example, paper diapers), toilet training pants, incontinence pads, sanitary products (sanitary napkins, tampons, and the like), sweat pads, pet sheets, portable toilet members, animal excrement treatment materials, and the like.

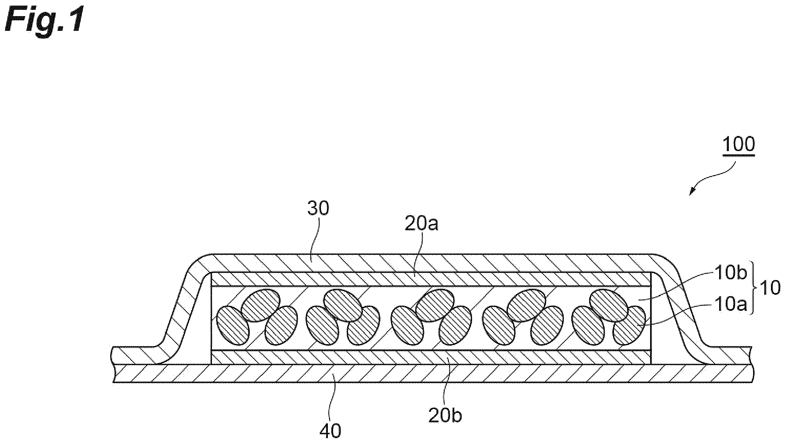

Fig. 1 is a cross-sectional view showing an example of an absorbent article. An absorbent article 100 shown in Fig. 1 includes an absorber 10, core wraps 20a and 20b, a liquid permeable top sheet 30, and a liquid impermeable back sheet 40. In the absorbent article 100, the liquid impermeable back sheet 40, the core wrap 20b, the absorber 10, the core wrap 20a, and the liquid permeable top sheet 30 are laminated in this order. In Fig. 1, there is a portion shown to be a gap between the members, but the members may be in close contact with each other without the gap.

The absorber 10 has water-absorbent resin particles 10a and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b.

The core wrap 20a and the core wrap 20b each have, for example, a main surface having the same size as that of the absorber 10. By using the core wraps, it is possible to maintain shape retainability of the absorber and prevent the water-absorbent resin particles and the like constituting the absorber from falling off and flowing. Examples of the core wraps include non-woven fabrics, woven fabrics, tissues, synthetic resin films having liquid permeation holes, net-like sheets having a mesh, and the like, of which tissues obtained by wet-type molding pulverized pulp are preferable from the viewpoint of economic efficiency.

The liquid permeable top sheet 30 is disposed on the outermost part on a side from which an absorption target liquid is infiltrated. The liquid permeable top sheet 30 is disposed on the core wrap 20a in a state of being in contact with the core wrap 20a. The liquid impermeable back sheet 40 is disposed on the outermost part on a side opposite to the liquid permeable top sheet 30, in the absorbent article 100. The liquid impermeable back sheet 40 is disposed below the core wrap 20b in a state of being in contact with the core wrap 20b. The liquid permeable top sheet 30 and the liquid impermeable back sheet 40 each have, for example, a main surface wider than the main surface of the absorber 10, and outer edges of the liquid permeable top sheet 30 and the liquid impermeable back sheet 40 respectively extend around the absorber 10 and the core wraps 20a and 20b.

Examples of the liquid permeable top sheet 30 include non-woven fabrics, porous sheets, and the like. Examples of non-woven fabrics include thermal bonded non-woven fabrics, air through non-woven fabrics, resin bonded non-woven fabrics, spunbond non-woven fabrics, melt-blown non-woven fabrics, spunbond/melt-blown/spunbond non-woven fabrics, airlaid non-woven fabrics, spunlace non-woven fabrics, point-bonded non-woven fabrics, and the like. Among them, thermal bonded non-woven fabrics, air through non-woven fabrics, spunbond non-woven fabrics, and spunbond/melt-blown/spunbond non-woven fabrics are preferably used.

As constituent materials for the liquid permeable top sheet 30, it is possible to use resins or fibers known in the technical field. Examples thereof include polyolefins such as polyethylene (PE) and polypropylene (PP); polyesters such as polyethylene terephthalate (PET), polytrimethylene terephthalate (PTT), and polyethylene naphthalate (PEN); polyamides such as nylon; rayon; other synthetic resins or synthetic fibers; and fibers such as cotton, silk, hemp, and pulp (cellulose), from the viewpoint of liquid permeability, flexibility, and strength when the liquid permeable top sheet 30 is used in an absorbent article. As the constituent material, synthetic fibers are preferably used from the viewpoint of increasing strength of the liquid permeable top sheet 30. Among them, polyolefins and polyesters are preferable. These materials may be used alone or in combination of two or more materials.

It is desirable that a non-woven fabric used for the liquid permeable top sheet 30 have appropriate hydrophilicity from the viewpoint of improving liquid absorption performances of the absorbent article. From this viewpoint, a non-woven fabric having a hydrophilicity of 5 to 200 is preferable, and a non-woven fabric having a hydrophilicity of 10 to 150 is more preferable, where the hydrophilicity is measured according to "Hydrophilicity of Non-woven fabric" (in accordance with Pulp and Paper Test Method No. 68 (2000)) disclosed in PCT International Publication No. WO2011/086843. Among the above-mentioned non-woven fabrics, a non-woven fabric having such a hydrophilicity may be formed of a material, such as rayon fibers, which shows an appropriate hydrophilicity by itself; or may be formed of fibers obtained by hydrophilizing hydrophobic chemical fibers such as polyolefin fibers and polyester fibers by a known method and imparting an appropriate hydrophilicity thereto.

Examples of methods of hydrophilizing chemical fibers include a method of obtaining a non-woven fabric by a spunbond technique using a mixture in which a hydrophilizing agent is added to hydrophobic chemical fibers in a spunbond non-woven fabric, a method of using a hydrophilizing agent when producing a spunbond non-woven fabric from hydrophobic chemical fibers, and a method of obtaining a spunbond non-woven fabric from hydrophobic chemical fibers, and thereafter impregnating the spunbond non-woven fabric with a hydrophilizing agent. As the hydrophilizing agent, the following examples are used: anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like.

It is preferable that a non-woven fabric used for the liquid permeable top sheet 30 be moderately bulky and have a large fabric weight per unit area from the viewpoint of imparting favorable liquid permeability, flexibility, strength, and cushioning properties to an absorbent article, and accelerating a liquid permeation rate of an absorbent article. A fabric weight per unit area of the non-woven fabric is preferably 5 to 200 $g/m^2$, is more preferably 8 to 150 $g/m^2$, and is even more preferably 10 to 100 $g/m^2$. Furthermore, a thickness of the non-woven fabric is preferably 20 to 1,400 $\mu$m, is more preferably 50 to 1,200 $\mu$m, and is even more preferably 80 to 1,000 $\mu$m.

The liquid impermeable back sheet 40 prevents a liquid absorbed by the absorber 10 from leaking to the outside from the back sheet 40 side. For the liquid impermeable back sheet 40, it is possible to use liquid impermeable films mainly composed of polyolefin resins such as polyethylene (PE) and polypropylene (PP); breathable resin films; composite films in which a breathable resin film is bonded to a non-woven fabric such as spunbond non-woven fabric and spunlace non-woven fabric; spunbond/melt-blown/spunbond (SMS) non-woven fabrics in which a water-resistant melt blown non-woven fabric is sandwiched between high-strength spunbond non-woven fabrics; and the like. For the back sheet 40, it is possible to use a resin film having a fabric weight per unit area of 10 to 50 $g/m^2$ and mainly made of low-density polyethylene (LDPE) resin from the viewpoint of ensuring flexibility so as not to impair a sensation of wearing the absorbent article. Furthermore, in a case where a breathable material is used, dampness generated when wearing the absorbent article is reduced, and thereby discomfort to a wearer can be reduced.

A magnitude relationship between the absorber 10, the core wraps 20a and 20b, the liquid permeable top sheet 30, and the liquid impermeable back sheet 40 is not particularly limited, and it is appropriately adjusted according to usage applications and the like of the absorbent article. Furthermore, a method of retaining the shape of the absorber 10 using the core wraps 20a and 20b is not particularly limited. The absorber may be sandwiched by a plurality of the core wraps as shown in Fig. 1, or the absorber may be covered with one core wrap.

The absorber 10 may be adhered to the liquid permeable top sheet 30. By adhering the absorber 10 to the liquid permeable top sheet 30, a liquid is more smoothly guided to the absorber, and thereby it becomes easy to obtain an absorbent article that is further better in preventing liquid leakage. In a case where the absorber 10 is sandwiched or covered by the core wrap, it is preferable that at least the core wrap and the liquid permeable top sheet 30 be adhered to each other, and it is more preferable that the core wrap and the absorber 10 be adhered to each other in addition to the adhesion of the core wrap and the liquid permeable top sheet 30. Examples of methods of adhesion include a method of adhesion by applying a hot melt adhesive to the liquid permeable top sheet 30 in shapes such as a vertical stripe shape and a spiral shape at predetermined intervals in a width direction; a method of adhesion using a water-soluble binder selected from starch, carboxymethyl cellulose, polyvinyl alcohol, polyvinylpyrrolidone, and other water-soluble polymers; and the like. Furthermore, a method of adhesion by thermal bonding may be adopted in a case where the absorber 10 contains heat-sealing synthetic fibers.

The present invention further provides a method for measuring a feeding retention rate of the water-absorbent resin particles which is represented by the following formula.

$$\text{Feeding retention rate (\%)} = (\text{feeding rate through swollen gel/feeding rate through dry powder}) \times 100$$

The feeding rate through a dry powder is measured by the method including the following steps (A), (B), and (C) in this order.

(A) 0.3 g of the water-absorbent resin particles is uniformly dispersed in a cylindrical container with an inner diameter of 26 mm having a mesh-like bottom part.

(B) A measurement unit is formed by inserting a cylindrical small-sized container with an outer diameter of 25 mm and an inner diameter of 19 mm having a mesh-like bottom part into the cylindrical container.

(C) A feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding.

The feeding rate through a swollen gel is measured by the method including the above-described steps (A) and (B), and further including the following steps (D) and (E) in this order.

(D) The water-absorbent resin particles in the measurement unit are swollen by immersing the bottom part side of the measurement unit in 40 g of physiological saline in the container for 30 minutes.

(E) A feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

As the feeding retention rate of the water-absorbent resin particles used for an absorber becomes higher, occurrence of tearing of the absorber after liquid absorption tends to be inhibited more. Accordingly, the present invention can also be recognized as a method for inhibiting occurrence of tearing of an absorber containing water-absorbent resin particles after the absorber absorbs a liquid, the method including increasing the feeding retention rate of the water-absorbent resin particles which is measured by the above-described measurement method. The water-absorbent resin particles in which a feeding retention rate is high have better diffusibility for an absorbed liquid, and the absorber containing the water-absorbent resin particles tends to have better shape retainability. Accordingly, the above-described method can also be recognized as a method for improving diffusibility of the water-absorbent resin particles or a method for improving shape retainability of the absorber. A feeding retention rate of the water-absorbent resin particles can be performed by, for example, selecting production conditions for the water-absorbent resin particles so that uniformity of crosslinking in the water-absorbent resin particles is high.

**Examples**

Hereinafter, the present invention will be described in more detail with reference to examples, but the present invention is not limited to these examples.

<Production of water-absorbent resin particles>

[Example 1]

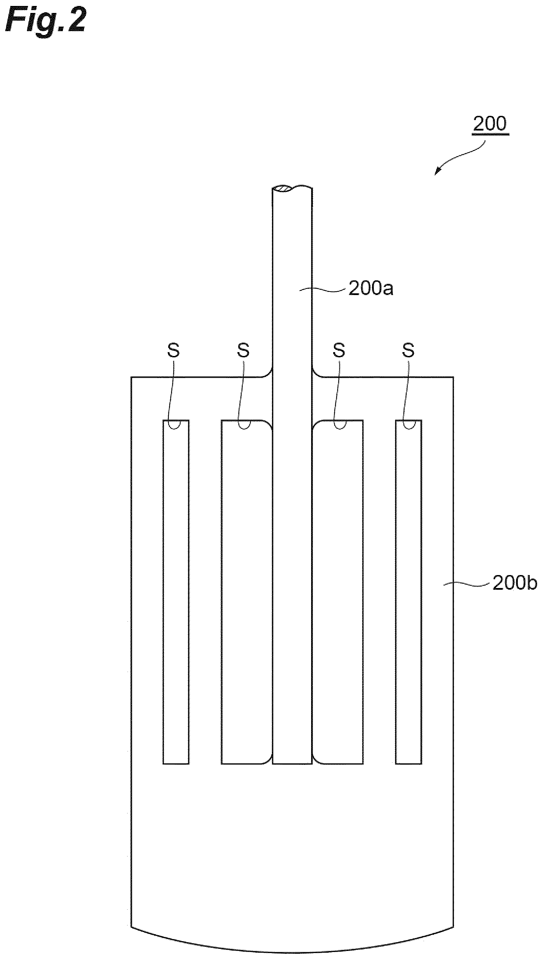

A cylindrical round-bottomed separable flask with the inner diameter of 11 cm and the internal volume of 2 L equipped with a reflux condenser, a dropping funnel, a nitrogen gas introduction tube, and a stirrer was prepared. The stirrer was equipped with a stirring blade (flat plate blade) 200 of which an outline is shown in Fig. 2. The stirring blade 200 includes a shaft 200a and a flat plate portion 200b. The flat plate portion 200b is welded to the shaft 200a and has a curved distal end. The flat plate portion 200b is formed with four slits S extending along an axial direction of the shaft 200a. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. Subsequently, 293 g of n-heptane as a hydrocarbon dispersion medium was put into the above-mentioned separable flask, and 0.736 g of a maleic anhydride-modified ethylene-propylene copolymer (HI-WAX 1105A, Mitsui Chemicals, Inc.) as a polymeric dispersant was added thereinto. Thereby, a mixture was obtained. The dispersant was dissolved by raising the temperature to 80°C while stirring the mixture, and then the mixture was cooled to 50°C.

Meanwhile, 92.0 g (1.03 moles) of an aqueous solution of 80.5% by mass acrylic acid as an ethylenically unsaturated monomer was put into a beaker with an internal capacity of 300 ml, and 147.7 g of an aqueous solution of 20.9% by mass sodium hydroxide was added dropwise thereto while cooling the beaker from the outside to perform neutralization to 75 mol%. Thereafter, 0.092 g of hydroxyethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.067 mmol) of potassium persulfate as a radical polymerization initiator, and 0.0046 g (0.026 mmol) of ethylene glycol diglycidyl ether as an internal crosslinking agent were added and dissolved. Thereby, a first stage aqueous liquid was prepared.

The prepared aqueous liquid was added into the reaction solution in the separable flask and stirred for 10 minutes. Then, a surfactant solution, in which 0.736 g of sucrose stearic acid ester (Mitsubishi-Chemical Foods Corporation, RYOTO Sugar Ester S-370, HLB: 3) as a surfactant was dissolved in 6.62 g of n-heptane by heating, was prepared. The surfactant solution was further added into the reaction solution. While stirring the reaction solution at 425 rpm as a rotation speed of the stirrer, the inside of the system was sufficiently replaced with nitrogen. Thereafter, the flask was immersed in a water bath at 70°C to raise its temperature, and polymerization was performed for 60 minutes. Thereby, a first stage polymerization slurry solution was obtained.

Meanwhile, 128.8 g (1.44 moles) of an aqueous solution of 80.5% by mass acrylic acid as an ethylenically unsaturated monomer was put into another beaker with an internal capacity of 500 ml, and 159.0 g of an aqueous solution of 27% by mass sodium hydroxide was added dropwise thereto while cooling the beaker from the outside to perform neutralization to 75 mol%. Thereafter, 0.129 g (0.476 mmol) of 2,2'-azobis(2-amidinopropane) dihydrochloride and 0.026 g (0.096 mmol) of potassium persulfate as a radical polymerization initiator, and 0.0116 g (0.067 mmol) of ethylene glycol diglycidyl ether as an internal crosslinking agent were added and dissolved. Thereby, a second stage aqueous liquid was prepared.

While stirring at 650 rpm as a rotation speed of the stirrer, the inside of the separable flask system was cooled to 25°C. Then, a total amount of the second stage aqueous liquid was added into the first stage polymerization slurry solution, and the inside of the system was replaced with nitrogen for 30 minutes. Thereafter, the flask was immersed in a water bath at 70°C again to raise its temperature, and a polymerization reaction was performed for 60 minutes. Thereby, a hydrous gel polymer was obtained.

Under stirring, 0.589 g of an aqueous solution of 45% by mass diethylenetriaminepentaacetic acid pentasodium was added to the hydrous gel polymer obtained after the second stage polymerization. Thereafter, the flask was immersed in an oil bath set to 125°C, and 207.9 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain polymer particles (dried product). These polymer particles were passed through a sieve having an opening of 850 $\mu$m, and 0.2% by mass of amorphous silica (Oriental Silicas Corporation, Tokusil NP-S) with respect to a mass of the polymer particles was mixed with the polymer particles. Thereby, 232.3 g of water-absorbent resin particles containing the amorphous silica was obtained. A median particle size of the water-absorbent resin particles was 361 $\mu$m.

[Example 2]

231.0 g of water-absorbent resin particles was obtained in the same manner as in Example 1 except that an amount of water extracted out of the system was changed to 224.3 g. The median particle size of the water-absorbent resin particles was 342 $\mu$m.

[Example 3]

232.1 g of water-absorbent resin particles was obtained in the same manner as in Example 1 except that an amount of water extracted out of the system was changed to 234.6 g. A median particle size of the water-absorbent resin particles was 355 $\mu$m.

[Example 4]

231.5 g of water-absorbent resin particles was obtained in the same manner as in Example 1 except that, in preparation of a first stage aqueous liquid, as a radical polymerization initiator, 2,2'-azobis(2-amidinopropane) dihydrochloride was not used, and a use amount of potassium persulfate was changed to 0.0736 g (0.272) mmol, and as an internal crosslinking agent, a use amount of ethylene glycol diglycidyl ether was changed to 0.010 g (0.057 mmol); in preparation of a second stage aqueous liquid, as a radical polymerization initiator, 2,2'-azobis(2-amidinopropane) dihydrochloride was not used, and a use amount of potassium persulfate was changed to 0.090 g (0.333 mmol); and an amount of water extracted out of the system was changed to 247.3 g. A median particle size of the water-absorbent resin particles was 359 $\mu$m.

[Example 5]

222.4 g of water-absorbent resin particles was obtained in the same manner as in Example 1 except that, in preparation of a first stage aqueous liquid, as an internal crosslinking agent, a use amount of ethylene glycol diglycidyl ether was changed to 0.010 g (0.057 mmol); and an amount of water extracted out of the system was changed to 238.5 g. A median particle size of the water-absorbent resin particles was 354 $\mu$m.

[Example 6]

229.8 g of water-absorbent resin particles was obtained in the same manner as in Example 1 except that, in preparation of a first stage aqueous liquid, as a radical polymerization initiator, 2,2'-azobis(2-amidinopropane) dihydrochloride was not used, and a use amount of potassium persulfate was changed to 0.0736 g (0.272 mmol), and as an internal crosslinking agent, a use amount of ethylene glycol diglycidyl ether was changed to 0.010 g (0.057 mmol); in preparation of a second stage aqueous liquid, as a radical polymerization initiator, 2,2'-azobis(2-amidinopropane) dihydrochloride was not used, and a use amount of potassium persulfate was changed to 0.090 g (0.334 mmol); and an amount of water extracted out of the system was changed to 257.6 g. A median particle size of the water-absorbent resin particles was 362 $\mu$m.

[Example 7]

224.0 g of water-absorbent resin particles was obtained in the same manner as in Example 1 except that an amount of water extracted out of the system was changed to 201.3 g; and a use amount of an aqueous solution of 2% by mass ethylene glycol diglycidyl ether as a surface crosslinking agent was changed to 6.62 g (0.761 mmol). The median particle size of the water-absorbent resin particles was 356 $\mu$m.

[Comparative Example 1]

A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was put into this separable flask, and 0.736 g of a maleic anhydride-modified ethylene-propylene copolymer (HI-WAX 1105A, Mitsui Chemicals, Inc.) as a polymeric dispersant was added thereinto. Thereby, a mixture was obtained. The dispersant was dissolved by raising the temperature to 80°C while stirring the mixture, and then the mixture was cooled to 50°C.

Meanwhile, 92.0 g (1.03 moles) of an aqueous solution of 80.5% by mass acrylic acid as an ethylenically unsaturated monomer was put into a beaker with an internal capacity of 300 ml, and 147.7 g of an aqueous solution of 20.9% by mass sodium hydroxide was added dropwise thereto while cooling the beaker from the outside to perform neutralization to 75 mol%. Thereafter, 0.092 g of hydroxyethyl cellulose (HEC AW-15F, manufactured by Sumitomo Seika Chemicals Co., Ltd.) as a thickener, 0.0736 g (0.272 mmol) of potassium persulfate as a radical polymerization agent, and 0.010 g (0.057 mmol) of ethylene glycol diglycidyl ether as an internal crosslinking agent were added and dissolved. Thereby, a first stage aqueous liquid was prepared.

The prepared aqueous liquid was added into the reaction solution in the separable flask and stirred for 10 minutes. Then, a surfactant solution, in which 0.736 g of sucrose stearic acid ester (Mitsubishi-Chemical Foods Corporation, RYOTO Sugar Ester S-370, HLB: 3) as a surfactant was dissolved in 6.62 g of n-heptane by heating, was prepared. The surfactant solution was further added into the reaction solution. While stirring the reaction solution at 550 rpm as a rotation speed of the stirrer, the inside of the system was sufficiently replaced with nitrogen. Thereafter, the flask was immersed in a water bath at 70°C to raise its temperature, and polymerization was performed for 60 minutes. Thereby, a first stage polymerization slurry solution was obtained.

Meanwhile, 128.8 g (1.44 moles) of an aqueous solution of 80.5% by mass acrylic acid as an ethylenically unsaturated monomer was put into another beaker with an internal capacity of 500 ml, and 159.0 g of an aqueous solution of 27% by mass sodium hydroxide was added dropwise thereto while cooling the beaker from the outside to perform neutralization to 75 mol%. Thereafter, as a radical polymerization initiator, 0.090 g (0.333 mmol) of potassium persulfate was added and dissolved to prepare a second stage aqueous liquid.

While stirring at 1,000 rpm as a rotation speed of the stirrer, the inside of the separable flask system was cooled to 25°C. Then, a total amount of the second stage aqueous liquid was added into the first stage polymerization slurry solution, and the inside of the system was replaced with nitrogen for 30 minutes. Thereafter, the flask was immersed in a water bath at 70°C again to raise its temperature, and a polymerization reaction was performed for 60 minutes. Thereafter, 0.580 g (0.067 mmol) of an aqueous solution of 2% by mass ethylene glycol diglycidyl ether was added as a crosslinking agent for post-polymerization crosslinking, and thereby a hydrous gel polymer was obtained.

Under stirring, 0.265 g of an aqueous solution of 45% by mass diethylenetriaminepentaacetic acid pentasodium was added to the hydrous gel polymer obtained after the second stage polymerization. Thereafter, the flask was immersed in an oil bath set to 125°C, and 259.4 g of water was extracted out of the system while n-heptane was refluxed by azeotropic distillation with n-heptane and water. Thereafter, 6.30 g (0.723 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain polymer particles (dried product). These polymer particles were passed through a sieve having an opening of 850 $\mu$m, and 0.5% by mass of amorphous silica (Oriental Silicas Corporation, Tokusil NP-S) with respect to a mass of the polymer particles was mixed with the polymer particles. Thereby, 230.8 g of water-absorbent resin particles containing the amorphous silica was obtained. A median particle size of the water-absorbent resin particles was 349 $\mu$m.

[Comparative Example 2]

230.8 g of water-absorbent resin particles was obtained in the same manner as in Comparative Example 1 except that, in preparation of a second stage aqueous liquid, 0.012 g (0.067 mmol) of ethylene glycol diglycidyl ether was added as an internal crosslinking agent; an aqueous solution of 2% by mass ethylene glycol diglycidyl ether for post-polymerization crosslinking was not used; an amount of water extracted out of the system was changed to 278.9 g; a use amount of an aqueous solution of 2% by mass ethylene glycol diglycidyl ether as a surface crosslinking agent was changed to 4.42 g (0.507 mmol); and a blending amount of amorphous silica with respect to polymer particles was changed to 0.2% by mass. A median particle size of the water-absorbent resin particles was 358 $\mu$m.

[Comparative Example 3]

230.3 g of water-absorbent resin particles was obtained in the same manner as in Comparative Example 1 except that, in preparation of a first stage aqueous liquid, a use amount of ethylene glycol diglycidyl ether as an internal crosslinking agent was changed to 0.011 g (0.063 mmol); in preparation of a second stage aqueous liquid, 0.013 g (0.075 mmol) of ethylene glycol diglycidyl ether ethylene glycol diglycidyl ether was added as an internal crosslinking agent; a crosslinking agent for post-polymerization crosslinking was not used; an amount of water extracted out of the system was changed to 262.6 g; a use amount of an aqueous solution of 2% by mass ethylene glycol diglycidyl ether as a surface crosslinking agent was changed to 9.93 g (1.14 mmol); and amorphous silica was not mixed. A median particle size of the water-absorbent resin particles was 349 $\mu$m.

The obtained water-absorbent resin particles were evaluated for a feeding retention rate, a water retention amount for physiological saline, a median particle size, a value of non-pressurization DW after 3 minutes, and absorber tearing properties by the following method. The physiological saline used in the present example was an aqueous solution of 0.9% by mass NaCl.

<Measurement of feeding retention rate>

[Measurement of feeding rate through swollen gel]

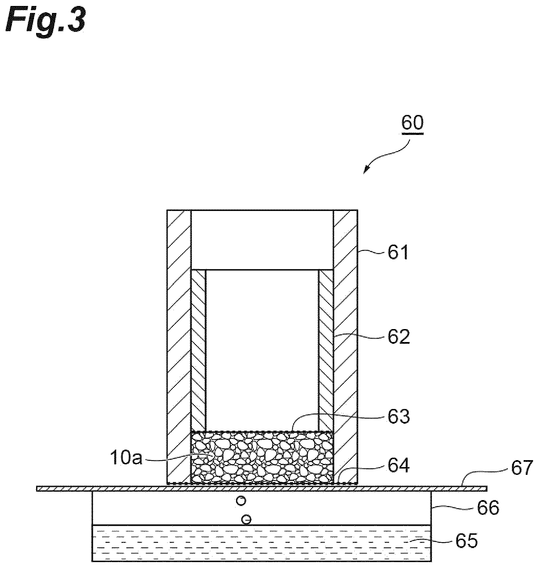

Measurement of a feeding rate through a swollen gel was performed using instruments shown in Fig. 3. A measurement unit 60 has an acrylic resin cylindrical container 61 having a nylon mesh sheet 64 with 250 mesh at a bottom part, and an acrylic resin cylindrical small-sized container 62 which also has a nylon mesh sheet 63 at a bottom part and is inserted inside the cylindrical container 61. The cylindrical container 61 has an inner diameter of 26 mm, an outer diameter of 40 mm, and a height of 140 mm. The cylindrical small-sized container 62 has an inner diameter of 19 mm, an outer diameter of 25 mm, a height of 120 mm, and a mass of 30 g. The cylindrical small-sized container 62 can be moved up and down inside the cylindrical container 61 without resistance.

A feeding rate through a swollen gel was measured as follows. 0.3 g of water-absorbent resin particles 10a was uniformly dispersed in the cylindrical container 61, the cylindrical small-sized container 62 was inserted from above, the water-absorbent resin particles 10a were sandwiched between the nylon mesh sheet 63 and the nylon mesh sheet 64, and thereby the measurement unit 60 having the water-absorbent resin particles 10a in a dry state was formed.

Separately, a petri dish containing 40 ± 0.1 g of physiological saline was prepared, and the bottom part side of the measurement unit 60 was immersed in the physiological saline for 30 minutes to cause the water-absorbent resin particles 10a to absorb the physiological saline and to be saturated and swollen. Thereby, the measurement unit 60 having a swollen gel was obtained.

A wire mesh 67 having a size of 10 cm square and an opening of 2 mm was placed on a petri dish 66 which has an inner diameter of about 90 mm and for which an empty mass (Wa) had been measured in advance. The measurement unit 60 containing the above-mentioned swollen gel was placed on the wire mesh 67. 20 g of physiological saline 65 was added into the measurement unit 60 at a time from the upper part of the cylindrical small-sized container 62. A mass (Wb) of the petri dish 66 containing the physiological saline 65 that had passed through the swollen gel for 1 minute from the start of the addition was measured, and a feeding rate (g/min) was obtained by the following formula. The results are shown in Table 1.

$$\text{Feeding rate (g/min)} = Wb - Wa$$

[Measurement of feeding rate through dry powder]

For measurement of a feeding rate through a dry powder, water-absorbent resin particles in a dry state which were stored at 25°C and a humidity of 45% (RH) were used. Measurement of a feeding rate was performed at 25°C and a humidity of 45% (RH). In the same manner as in the measurement of the feeding rate through a swollen gel, a measurement unit 60 having water-absorbent resin particles 10a in a dry state was formed. From the upper part of the cylindrical small-sized container 62, physiological saline was added into the measurement unit 60 at a constant rate of 60 ml/min for 1 minute using an automatic pump (INTEGRA DOSE IT P910). A liquid, which passed through the water-absorbent resin particles 10a in the measurement unit 60 and flowed out from the lower part of the measurement unit 60, was stored in another container, and a mass of the stored liquid was measured. Thereby, an amount (feeding rate (g/min)) of the liquid passed through the dry powder for 1 minute from the start of the addition was obtained. The results are shown in Table 1.

A feeding retention rate was calculated from the measured feeding rate through a swollen gel and feeding rate through a dry powder. The results are shown in Table 1.

$$\text{Feeding retention rate (\%)} = (\text{feeding rate through swollen gel/feeding rate through dry powder}) \times 100$$

<Measurement of water retention amount for physiological saline>

A cotton bag (cotton broadcloth No. 60, 100 mm in width × 200 mm in length) into which 2.0 g of the water-absorbent resin particles had been weighed was placed in a beaker having a capacity of 500 ml. 500 g of physiological saline was poured into the cotton bag containing the water-absorbent resin particles at a time so that a lump could not be produced. The upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wc (g) of the dehydrated cotton bag containing the swollen gel was measured. By performing the same operation without addition of the water-absorbent resin particles, a mass Wd (g) of an empty cotton bag upon moisturizing was measured, and a water retention amount for physiological saline was calculated by the following formula. The results are shown in Table 1.

$$\text{Water retention amount for physiological saline (g/g)} = [Wc - Wd]/2.0$$

<Measurement of median particle size (particle size distribution)>

50 g of the water-absorbent resin particles was used for measuring a median particle size (particle size distribution). JIS standard sieves were combined in the following order from the top: a sieve having an opening of 850 μm, a sieve having an opening of 500 μm, a sieve having an opening of 425 μm, a sieve having an opening of 300 μm, a sieve having an opening of 250 μm, a sieve having an opening of 180 μm, a sieve having an opening of 150 μm, and a saucer.

The water-absorbent resin particles were fed to the topmost sieve among the combination of the sieves, and using a Ro-Tap shaker (manufactured by Iida-seisakusho Japan Corporation), classification was performed according to JIS Z 8815 (1994). After the classification, a mass of the water-absorbent resin particles remaining on each of the sieves was calculated as a mass percentage with respect to a total amount to determine a particle size distribution. By integrating values on the sieves in descending order of the particle sizes with regard to the particle size distribution, a relationship between the opening of the sieve and the integrated value of mass percentages of the water-absorbent resin particles remaining on the sieve was plotted on a log-probability paper. The plotted points on the probability paper were connected with straight lines, and a particle size corresponding to 50% by mass of the integrated mass percentage was taken as a median particle size.

<Measurement of non-pressurization DW>

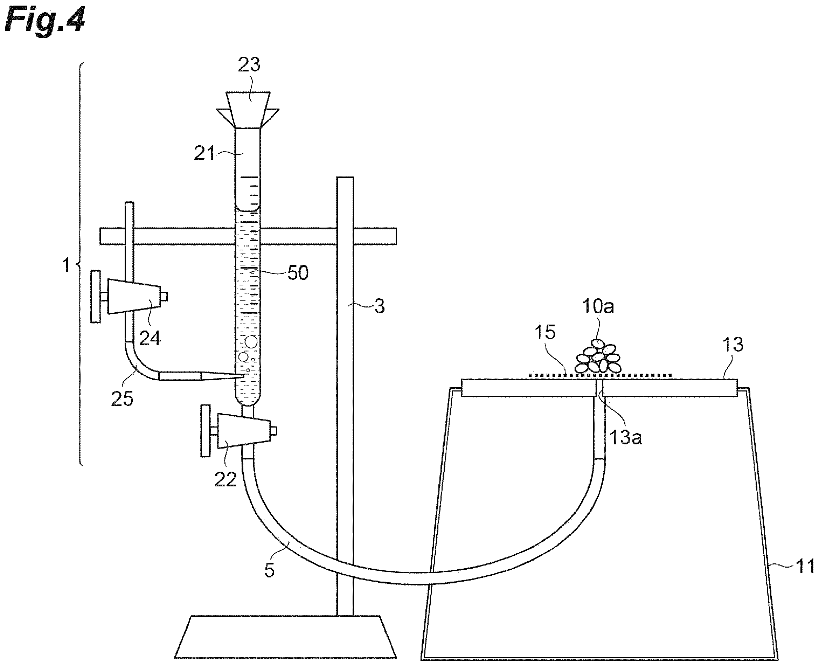

Non-pressurization DW of the water-absorbent resin particles was measured using a measurement device shown in Fig. 4. The measurement was carried out five times for one type of water-absorbent resin particles, and an average value of three measurement values excluding a minimum value and a maximum value was obtained.

The measurement device has a burette unit 1, a conduit 5, a measurement table 13, a nylon mesh sheet 15, a stand 11, and a clamp 3. The burette unit 1 has a burette tube 21 on which a scale is engraved, a rubber stopper 23 for sealing an opening at an upper part of the burette tube 21, a cock 22 connected to a distal end of a lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having a diameter of 2 mm and formed in the central portion of the measurement table 13, and is supported by the height-variable stand 11. The through hole 13a of the measurement table 13, and the cock 22 of the burette unit 1 are connected by the conduit 5. An inner diameter of the conduit 5 is 6 mm.

The measurement was performed in the environment of a temperature of 25°C and a humidity of 60 $\pm$ 10%. First, the cock 22 and the cock 24 of the burette unit 1 were closed, and physiological saline 50 that had been adjusted to 25°C was put into the burette tube 21 through the opening at the upper part of the burette tube 21. A concentration (0.9% by mass) of the physiological saline is a concentration based on a mass of an aqueous solution of NaCl. The opening of the burette tube 21 was sealed with the rubber stopper 23, and then the cock 22 and the cock 24 were opened. The inside of the conduit 5 was filled with the 0.9% by mass saline solution 50 to prevent air bubbles from entering. A height of the measurement table 13 was adjusted so that a height of a water surface of the physiological saline, which had reached the inside of the through hole 13a, was the same as a height of an upper surface of the measurement table 13. After the adjustment, the height of the water surface of the physiological saline 50 in the burette tube 21 was read by the scale on the burette tube 21, and this position was defined as a zero point (value read at 0 seconds).

The nylon mesh sheet 15 (100 mm $\times$ 100 mm, 250 mesh, thickness about 50 $\mu$m) was laid in the vicinity of the through hole 13a on the measurement table 13, and a cylinder having an inner diameter of 30 mm and a height of 20 mm was placed on the central portion of the nylon mesh sheet. 1.00 g of water-absorbent resin particles 10a were uniformly dispersed in this cylinder. Thereafter, the cylinder was carefully removed to obtain a sample in which the water-absorbent resin particles 10a were dispersed in a circle shape in the central portion of the nylon mesh sheet 15. Then, the nylon mesh sheet 15 on which the water-absorbent resin particles 10a were placed was moved at a high speed to the extent that the water-absorbent resin particles 10a did not dissipate so that the center of the nylon mesh sheet was at the position of the through hole 13a, and the measurement was started. A timing when air bubbles were first introduced from the air introduction tube 25 into the burette tube 21 was defined as a start of water absorption (0 seconds).

An amount of decrease in the physiological saline 50 in the burette tube 21 (that is, an amount of the physiological saline absorbed by the water-absorbent resin particles 10a) was sequentially read by units of 0.1 ml, and a reduction in weight We (g) of the physiological saline 50 was read 3 minutes after the start of water absorption by the water-absorbent resin particles 10a. A value of non-pressurization DW after 3 minutes was obtained from We by the following formula. The non-pressurization DW is a water absorption amount per 1.00 g of the water-absorbent resin particles 10a. The results are shown in Table 1.

$$\text{Value of non-pressurization DW after 3 minutes (ml/g)} = \text{We}/1.00$$

<Measurement of absorber tearing properties>

[Production of absorbent article for evaluation]

10 g of the water-absorbent resin particles and 6.67 g of pulverized pulp were uniformly mixed by air papermaking using a Padformer manufactured by OTEC Co., Ltd., and thereby an absorber having a size of 40 cm $\times$ 12 cm was produced. 0.4 g of ion-exchanged water was sprayed with a spray on the absorber placed on a tissue paper having a size of 42 cm $\times$ 14 cm and a fabric weight per unit area of 16 g/m$^2$, another tissue of 40 cm $\times$ 12 cm was placed on this absorber, and thereby a laminate was produced. Next, a wire mesh having a size of 62 cm $\times$ 22 cm and having an opening of 2 mm was placed on the laminate, and the laminate was pressed at a pressure of 0.141 MPa using a press machine (small-sized air type press machine, Imoto Machinery Co., Ltd.). After the pressing, the wire mesh was removed from the laminate.

Portions at a distance of 15 cm away in a longitudinal direction from the center portion of the pressed laminate were each cut with a roll cutter so that a length of became 30 cm. The laminate was sandwiched between two polyethylene air-through type porous liquid permeable sheets (hereinafter, also referred to as "liquid permeable sheets") having a fabric weight per unit area of 22 g/m$^2$ and a size of 34 cm $\times$ 14 cm. Four sides of the two liquid permeable sheets sandwiching the laminate were crimped by a heat sealer (Fuji Impulse Sealer, model number: FI-450-5 type, manufactured by FUJI IMPULSE CO., LTD.), and thereby an absorbent article 100' for evaluation was obtained. At the time of crimping, a portion of the tissue protruded from the absorber was crimped by sandwiching the protruding tissue with the liquid permeable sheets.

[Preparation of test solution]

An appropriate amount of distilled water was put into a 10 L container, and 100 g of sodium chloride, 3.0 g of a calcium chloride dihydrate, and 6.0 g of a magnesium chloride hexahydrate were added and dissolved therein. Then, 0.25 g of polyoxyethylene nonylphenyl ether was added thereto, and distilled water was further added so that a total mass became 10 kg. Furthermore, the mixed solution was colored with a small amount of Blue No. 1, and thereby a test solution for measuring tearing properties was prepared.

[Measurement 1 of absorber tearing time]

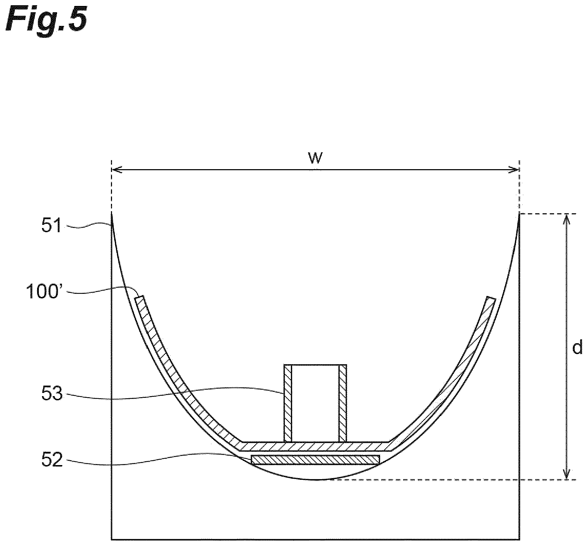

An absorber tearing time was measured in a room at a temperature of 25°C ± 2°C using a U-shaped instrument 51 (depth 14 cm, made of acryl) shown in Fig. 5. In the U-shaped instrument 51, an opening width w was 21 cm, and a depth d was 18.5 cm. The U-shaped instrument 51 was used so that the opening was upward. A paperboard 52 with 14 cm × 6.5 cm was placed on a U-shaped bottom portion to form a flat bottom portion with width 6.5 cm × depth 14 cm. An absorbent article 100' for evaluation was placed on the U-shaped instrument 51 by matching the center of the absorbent article 100' for evaluation with the center of the paperboard 52, and causing a longitudinal direction of the absorbent article 100' for evaluation to be along the curve of the U-shaped instrument 51. A cylindrical cylinder 53 having an inner diameter of 2 cm, an outer diameter of 3 cm, a height of 6.5 cm, and a mass of 60 g was put on the center of the absorbent article 100' for evaluation, and 80 ml of the above test solution adjusted to 25°C ± 1°C was added into the cylinder 53. During the addition of the test solution, the addition was continued to maintain a height of a liquid level of the test solution at about 5 cm from the bottom of the cylinder.
After the entire test solution added into the cylinder 53 was absorbed by the absorbent article 100' for evaluation, the cylinder 53 on the absorbent article 100' for evaluation was removed. The cylinder 53 was placed again on the center of the absorbent article 100' for evaluation, which was placed on the U-shaped instrument 51, 3 minutes after start of adding the test solution, and 80 ml of the test solution (second time) was added and absorbed by the absorbent article 100' for evaluation in the same manner as described above.
The absorbent article 100' for evaluation was placed on a paperboard (fabric weight per unit area 3,500 g/m$^2$) having the same size as that of the absorbent article 100' for evaluation 6 minutes after start of the first addition of the test solution. Both ends in a longitudinal direction were bonded with an adhesive tape to fix the absorbent article 100' for evaluation to the paperboard. The fixed absorbent article 100' for evaluation was put into a transparent polyethylene bag (40 cm × 28 cm, thickness 0.04 mm, Unipack, manufactured by SEISANNIPPONSHALTD., K-4) which was attached with a zipper and in which a 1-cm cut was made at three points to remove air. The absorbent article 100' for evaluation was put into the above-mentioned transparent bag so that the corner of the bag and the corner of the absorbent article 100' for evaluation were matched with each other. The remaining part of the bag was folded back and fixed with an adhesive tape, and the entire bag was filled so that it has approximately the same size as the absorbent article 100' for evaluation.
The filled absorbent article 100' for evaluation was centrifuged 11 minutes after start of the first addition of the test solution at a centrifugal force of 27.5 G (rotation speed: 405 r/min). The centrifugation was performed in a state in which the filled absorbent article 100' for evaluation was fixed with a packing tape on a turntable with a diameter of 30 cm so that the surface of the absorbent article 100' for evaluation was perpendicular to a rotation shaft, and the rotation shaft was at the center of the absorbent article 100' for evaluation.
The centrifugation was interrupted every 1 minute from the start of centrifugation until 4 minutes, and every 2 minutes after 4 minutes from the start of centrifugation, and the presence or absence of tearing of the absorber after liquid absorption was visually confirmed. A total centrifugation time until tearing was recognized was defined as a tearing time. It can be determined that as a tearing time becomes longer, performances are more favorable. In a case where the measurement was carried out for up to 20 minutes but tearing of the absorber was not recognized within this time, it was evaluated as "20 minutes or longer." The results are shown in Table 1.

[Measurement 2 of absorber tearing time]

Tearing properties of the absorber obtained in the example was evaluated in the same manner as in the above-described measurement 1 of absorber tearing time except that 80 ml of the test solution (third time) was further added after 3 minutes in the same manner after the second addition of the test solution, and the subsequent operations were delayed by 3 minutes. The results are shown in Table 1.

[Table 1]

| | Water retention amount [g/g] | Value of non-pressurization DW after 3 minutes [ml/g] | Feeding rate through dry powder [g/min] | Feeding rate through swollen gel [g/min] | Feeding retention rate [%] | Absorber tearing time [min] | |
|---|---|---|---|---|---|---|---|
| | | | | | | Measurement 1 (addition of 2 times) | Measurement 2 (addition of 3 times) |
| Example 1 | 34 | 27 | 23.7 | 10.1 | 42 | 20 or longer | 20 or longer |
| Example 2 | 44 | 15 | 11.1 | 4.3 | 38 | 20 or longer | 6 |
| Example 3 | 51 | 20 | 9.0 | 3.1 | 34 | 20 or longer | 2 |
| Example 4 | 35 | 32 | 16.2 | 2.1 | 13 | 20 or longer | 1 |
| Example 5 | 40 | 49 | 8.3 | 1.1 | 13 | 20 or longer | 1 |
| Example 6 | 41 | 27 | 19.8 | 1.9 | 10 | 20 or longer | 1 |
| Example 7 | 28 | 18 | 33.3 | 18.5 | 56 | 20 or longer | 20 or longer |
| Comparative Example 1 | 41 | 49 | 24.1 | 1.4 | 6 | 6 | - |
| Comparative Example 2 | 50 | 19 | 15.6 | 0.1 | 1 | 1 | - |
| Comparative Example 3 | 40 | 12 | 22.0 | 1.8 | 8 | 10 | - |

In the absorber, which was formed using the water-absorbent resin particles of the examples in which a feeding retention rate was constant or higher, a tearing time was 20 minutes or longer in the measurement of tearing properties in the measurement 1 (where the test solution was added twice), and it was confirmed that occurrence of tearing of the absorber after liquid absorption was inhibited. In the measurement of tearing properties in the measurement 2 which was under the stricter conditions (where the test solution was added 3 times), it was confirmed that tearing properties were further inhibited in Examples 1 to 3 and 7, particularly in Examples 1 and 7.

**Reference Signs List**

| | |
|---|---|
| 1 | burette unit, |
| 3 | clamp, |
| 5 | conduit, |
| 10 | absorber, |
| 10a | water-absorbent resin particle, |
| 10b | fiber layer, |
| 11 | stand, |
| 13 | measurement table, |
| 13a | through hole, |
| 15, 63, 64 | nylon mesh sheet, |
| 20a, 20b | core wrap, |
| 21 | burette tube, |
| 22 | cock, |
| 23 | rubber stopper, |
| 24 | cock, |
| 25 | air introduction tube, |
| 30 | liquid permeable top sheet, |
| 40 | liquid impermeable back sheet, |
| 50 | physiological saline, |
| 51 | U-shaped instrument, |
| 52 | paperboard, |
| 53 | cylindrical cylinder, |
| 60 | measurement unit, |
| 61 | cylindrical container, |
| 62 | cylindrical small-sized container, |
| 65 | physiological saline, |
| 66 | petri dish, |
| 67 | wire mesh, |
| 100, 100' | absorbent article, |
| 200 | stirring blade, |
| 200a | shaft, |
| 200b | flat plate portion, |
| S | slit. |

**Claims**

1. Water-absorbent resin particles having a feeding retention rate of 10% or more,
   the feeding retention rate being represented by the following formula using a feeding rate through a dry powder measured by a method including the following (A), (B), and (C) in this order, and a feeding rate through a swollen gel measured by a method including the following (A), (B), (D), and (E) in this order,

$$\text{feeding retention rate (\%)} = (\text{feeding rate through swollen gel/feeding rate through dry powder}) \times 100,$$

(A) 0.3 g of the water-absorbent resin particles is uniformly dispersed in a cylindrical container with an inner diameter of 26 mm having a mesh-like bottom part,

(B) a measurement unit is formed by inserting a cylindrical small-sized container with an outer diameter of 25 mm and an inner diameter of 19 mm having a mesh-like bottom part into the cylindrical container,

(C) a feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding,

(D) the water-absorbent resin particles in the measurement unit are swollen by immersing the bottom part side of the measurement unit in 40 g of physiological saline in the container for 30 minutes, and

(E) a feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

2. The water-absorbent resin particles according to claim 1, wherein the feeding rate through a swollen gel is 2.5 g/min or more.

3. An absorber comprising the water-absorbent resin particles according to claim 1 or 2.

4. An absorbent article comprising the absorber according to claim 3.

5. The absorbent article according to claim 4, wherein the absorbent article is a diaper.

6. A method for measuring a feeding retention rate of water-absorbent resin particles represented by the following formula, the method comprising:

measuring a feeding rate through a dry powder by a method including the following (A), (B), and (C) in this order; and

measuring a feeding rate through a swollen gel by a method including the following (A), (B), (D), and (E) in this order,

$$\text{feeding retention rate } (\%) = (\text{feeding rate through swollen gel/feeding rate through dry powder}) \times 100,$$

(A) 0.3 g of the water-absorbent resin particles is uniformly dispersed in a cylindrical container with an inner diameter of 26 mm having a mesh-like bottom part,

(B) a measurement unit is formed by inserting a cylindrical small-sized container with an outer diameter of 25 mm and an inner diameter of 19 mm having a mesh-like bottom part into the cylindrical container,

(C) a feeding rate (g/min) of physiological saline through a dry powder is obtained by adding physiological saline into the cylindrical small-sized container of the measurement unit at a constant rate of 60 ml/min, and measuring an amount of the physiological saline flowing out from the bottom part of the measurement unit within 1 minute from start of the adding,

(D) the water-absorbent resin particles in the measurement unit are swollen by immersing the bottom part side of the measurement unit in 40 g of physiological saline in the container for 30 minutes, and

(E) a feeding rate (g/min) of physiological saline through a swollen gel is obtained by adding 20 g of physiological saline into the cylindrical small-sized container of the measurement unit at a time, and measuring an amount of the physiological saline flowing out from the bottom part of the measurement unit within 1 minute from start of the adding.

7. A method for producing water-absorbent resin particles, the method comprising:
sorting out water-absorbent resin particles having the feeding retention rate of 10% or more, the feeding retention rate being measured by the method according to claim 6.

8. A method for inhibiting occurrence of tearing of an absorber containing water-absorbent resin particles after the absorber absorbs a liquid, the method comprising:
increasing the feeding retention rate of the water-absorbent resin particles measured by the method according to claim 6.

*Fig.1*

*Fig.2*

# Fig.3

# Fig.4

EP 3 936 225 A1

**Fig.5**

## INTERNATIONAL SEARCH REPORT

International application No.

PCT/JP2020/009507

### A. CLASSIFICATION OF SUBJECT MATTER

Int.Cl. B01J20/26(2006.01)i, A61F13/49(2006.01)i, A61F13/53(2006.01)i,
B01J20/28(2006.01)i
FI: B01J20/26D, B01J20/28Z, A61F13/53300, A61F13/49

According to International Patent Classification (IPC) or to both national classification and IPC

### B. FIELDS SEARCHED

Minimum documentation searched (classification system followed by classification symbols)
Int.Cl. B01J20/20-B01J20/34, A61F13/49, A61F13/53

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2005-344103 A (NIPPON SHOKUBAI CO., LTD.)<br>15.12.2005 (2005-12-15), claims 1, 6, paragraphs<br>[0094]-[0099], [0204], table 1 | 1-5<br>6-8 |
| X<br>A | JP 2010-540207 A (NIPPON SHOKUBAI CO., LTD.)<br>24.12.2010 (2010-12-24), claim 1, paragraphs<br>[0001], [0017], [0169]-[0174], [0202], table 1 | 1-5<br>6-8 |
| X<br>A | WO 2017/002972 A1 (NIPPON SHOKUBAI CO., LTD.)<br>05.01.2017 (2017-01-05), claims 1, 5, paragraphs<br>[0002], [0035], [0041], [0222]-[0226], [0302],<br>table 1 | 1-5<br>6-8 |
| X<br>A | EP 3412711 A1 (LG CHEM, LTD.) 12.12.2018 (2018-12-<br>12), paragraphs [0002]-[0004], [0016], [0096],<br>[0097] | 1-5<br>6-8 |
| A | JP 2018-164732 A (SDP GLOBAL CO., LTD.) 25.10.2018<br>(2018-10-25) | 1-8 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11.05.2020 | 19.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

International application No.

PCT/JP2020/009507

| | | |
|---|---|---|
| JP 2005-344103 A | 15.12.2005 | US 2008/0032888 A1<br>claims 1, 6,<br>paragraphs [0094]-[0106], [0213], table 1<br>WO 2005/108472 A1<br>KR 10-2007-0012731 A<br>CN 1965019 A |
| JP 2010-540207 A | 24.12.2010 | US 2011/0114881 A1<br>claim 1, paragraphs [0001], [0024],<br>[0209]-[0220], [0254], table 1<br>WO 2009/041731 A1<br>CN 101808727 A |
| WO 2017/002972 A1 | 05.01.2017 | US 2018/0161756 A1<br>claims 1, 5, paragraphs [0002],<br>[0130], [0131], [0141], [0142],<br>[0408]-[0413], page 23, table 1<br>EP 3318324 A1<br>CN 107847905 A<br>KR 10-2018-0022883 A |
| EP 3412711 A1 | 12.12.2018 | JP 2019-518815 A<br>US 2019/0085104 A1<br>WO 2018/124550 A1<br>KR 10-2018-0075309 A<br>CN 108884237 A |
| JP 2018-164732 A | 25.10.2018 | WO 2018/181277 A1<br>CN 110475610 A |

Form PCT/ISA/210 (patent family annex) (January 2015)

**EP 3 936 225 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014515987 W

- WO 2011086843 A

**Non-patent literature cited in the description**

- Hydrophilicity of Non-woven fabric. *Pulp and Paper Test Method,* 2000, (68